# EUROPÄISCHE PATENTSCHRIFT

(11) **EP 0 748 790 B1**
(45) Veröffentlichungstag und Bekanntmachung des Hinweises auf die Patenterteilung: **20.10.1999**
(21) Anmeldenummer: 96108856.4
(22) Anmeldetag: 03.06.1996
(51) Int. Cl.: C07C 209/36, B01J 23/62, B01J 21/18, B01J 23/44

(54) **Verfahren und Katalysator zur Herstellung von aromatischen Aminen durch Gasphasenhydrierung**
Process and catalyst for the preparation of aromatic amines by hydrogenation in the gas phase
Procédé et catalyseur pour la préparation d'amines aromatiques par hydrogénation en phase gazeuse

(30) Priorität: 14.06.1995 DE 19521587
(43) Veröffentlichungstag der Anmeldung: 18.12.1996
(73) Patentinhaber: BAYER AG, 51368 Leverkusen (DE)
(72) Erfinder: Langer, Reinhard, Dr., 47800 Krefeld (DE); Buysch, Hans-Josef, Dr., 47809 Krefeld (DE); Pentling, Ursula, Dr., 47239 Duisburg (DE)

(56) Entgegenhaltungen:
- EP-A- 0 011 090
- EP-A- 0 490 151
- EP-A- 0 494 568
- EP-A- 0 696 573
- DE-A- 2 042 368

## Beschreibung

Die vorliegende Erfindung betrifft ein verbessertes Verfahren zur Herstellung von Anilinen durch katalytische Hydrierung von Nitroaromaten in der Gasphase sowie ein für das Verfahren geeigneten, neuen Katalysator.

Aniline sind dem Fachmann als wichtige Zwischenprodukte, beispielsweise zur Herstellung von Farbstoffen, Polyurethanen und Pflanzenschutz-Produkten bekannt.

Zur Hydrierung von Nitrobenzol und anderen Nitroaromaten sind verschiedene Verfahrensweisen Stand der Technik, die aufgrund der großen freiwerdenden Reaktionsenthalpie alle in Reaktoren mit integrierten Wärmeträgersystemen durchgeführt werden: z.B. Hydrierungen in flüssiger Phase an suspendierten Katalysatoren, mit z.B. Pd-Katalysatoren wie in EP 476404 beschrieben oder an fluidisierten festen Katalysatoren wie z.B. in US 3136818 veröffentlicht, oder Hydrierungen in der Gasphase an ortsfest angeordneten Katalysatoren z.B. mit Pd-Trägerkatalysatoren wie in DE-A 2244401, 2849002 und 4039026 beschrieben.

In DE-A 2244401 und 2849002 werden Pd-Katalysatoren auf Aluminiumoxid-Trägern beschrieben, die als stationäre Katalysatorschüttungen in Wärmetauscherrohren unter Normaldruck bei Belastungen von weniger als 1g Nitrobenzol (NBz) /ml (Kat) h mit geringen Wasserstoff-Nitrobenzol-Verhältnissen betrieben werden. Es werden zwischen 6 und 11 Molen Wasserstoff pro Mol Nitrobenzol eingesetzt. Die Katalysatoren müssen ca. alle 1000 Stunden regeneriert werden. Die Verfahren werden in einem GHSV-Bereich zwischen 600 und 900 h⁻¹ betrieben.

In DE-A 4039026 werden Pd-Katalysatoren auf graphitischen Trägern beschrieben, die unter ähnlichen Bedingungen wie die Pd-Katalysatoren auf Aluminiumoxid betrieben werden. Die Katalysatoren zeigen bei Belastungen deutlich unter 1g (NBz) / ml (Kat) h und einem Wasserstoff-Nitrobenzol-Verhältnis von 14-26 Molen zu 1 Mol unvollständigen Umsatz. Im Kondensat werden zwischen 1000 und 4000 ppm Nitrobenzol, bezogen auf gebildetes Anilin, gefunden. Ferner schwanken die Selektivitäten bezüglich Anilin zwischen 99,1 und 99,6%. Das Verfahren wird für einen GHSV-Bereich zwischen ca. 2000 und 3150 h⁻¹ beschrieben.

Sowohl eine Vergrößerung der Nitroaromatenbelastung als auch ein Heraufsetzen des Wasserstoff-Nitroaromaten-Verhältnisses erhöht den Volumenstrom durch das Katalysatorbett und verkleinert damit die Verweilzeit am Kontakt. Beide Maßnahmen sollten demnach zu einer Vergrößerung des Nitroaromatendurchbruches, d.h. unvollständigen Umsatz führen.

Ein allgemeines Maß für diesen Gasstrom durch das Katalysatorbett ist die Gas Hourly Space Velocity (GHSV), angegeben in h⁻¹.

Schon geringe Mengen an Nitroaromaten in Anilinen führen aber zu deutlichen Verfärbungen des sonst farblosen aromatischen Amins und sind daher nicht erwünscht. Eine destillative Abtrennung der Nitroaromaten ist apparativ und energetisch aufwendig.

Bei all diesen Verfahrensvarianten muß die große anfallende Reaktionswärme einem technischen Reaktor über ein aufwendiges Wärmeträgersystem entzogen werden.

Hydrierungsverfahren in der Gasphase mit einfachen adiabaten Katalysatorschüttungen sind wegen des einfachen apparativen Aufbaus mit Reaktoren ohne integriertem Wärmetauschersystem besonders wirtschaftlich. Die große Exothermie der Nitrogruppenhydrierung stellt aber besondere Anforderungen an den Katalysator, der in einem weiten Temperaturbereich selektiv hydrieren muß. Ferner wird beim adiabaten Verfahren dem Eduktgemisch zusätzlich ein Wärmeträger, bei Hydrierungen i.a. Wasserstoff, zu gemischt, was zu sehr kurzen Verweilzeiten bzw. großen GHSVs führt. Damit muß der Katalysator neben seiner Selektivität in einem weiten Temperaturbereich sehr aktiv sein, um einen vollständigen Nitrobenzolumsatz auch bei kleinen Nitrobenzolbelastungen zu erzielen.

Aufgabe der Erfindung war es daher, einen Katalysator zu finden, der belastbarer ist als die bekannten Katalysatoren, d.h. bei großen GHSVs vollständigen Umsatz zeigt und in einfachen Reaktorkonstruktionen betrieben werden kann.

Weiter ist es wünschenswert, vom Anfahren des Katalysator bis zu dessen Desaktivierung durchgehend hohe Selektivitäten zu erzielen, so daß auf eine teure und apparativ aufwendige destillative Aufarbeitung des Anilinkondensates verzichtet werden kann.

Es wurde nun gefunden, daß man die Herstellung von aromatischen Aminen durch katalytische Hydrierung von Nitroaromaten in der Gasphase mit unerwartet langen Standzeiten durchführen kann und dabei ausgezeichnete Selektivitäten erzielt, wenn man die Hydrierung an Palladium-Blei-Katalysatoren auf Graphit oder graphitischem Kohlenstoff als Träger in Gegenwart großer Wasserstoffüberschüsse durchführt, wobei auf einen Wärmeaustrag aus dem Katalysatorbett mittels eines zusätzlichen Wärmeträgersystems verzichtet werden kann, d.h. es genügen einfache adiabate Reaktorkonstruktionen, wie sie für großtechnische Produktionen Stand der Technik sind, aber für die Nitrobenzolhydrierung bisher nicht eingesetzt werden konnten. Ferner kann aufgrund der durchgehend hohen Selektivität gegebenenfalls auf eine aufwendige destillative Aufarbeitung des kondensierten Anilines verzichtet werden.

Die Erfindung betrifft demnach ein Verfahren zur Herstellung aromatischer Amine der Formel 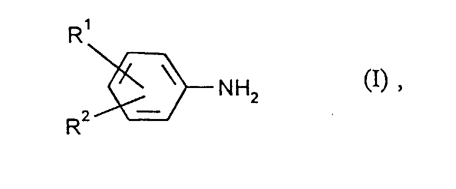 in der
R¹ und R² unabhängig voneinander Wasserstoff, Methyl oder Ethyl bedeuten, wobei R¹ zusätzlich Amino bedeuten kann,
   durch Hydrierung von Nitroaromaten der Formel 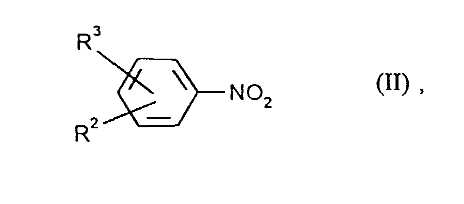 in der
R² und R³ unabhängig voneinander Wasserstoff, Methyl oder Ethyl bedeuten,
   wobei R³ zusätzlich Nitro bedeuten kann,
mit Wasserstoff an ortsfest angeordneten Katalysatoren in der Gasphase, wobei als Katalysator Palladium und Blei auf Graphit oder graphithaltigem Koks als Träger, der eine BET-Oberfläche von 0,2-10 m²/g hat, eingesetzt wird, das dadurch gekennzeichnet ist, daß der Katalysator einen Palladiumgehalt, bezogen auf das Gesamtgewicht des Katalysators, von 0,001 bis 7 Gew.-%, bevorzugt von 0,1 bis 6 Gew.-%, besonders bevorzugt von 0,5 bis 5 Gew.-%, ganz besonders bevorzugt von 1,5 bis 4 Gew.-%, hat und einen Bleigehalt von 0,1 bis 50 Gew.-%, bevorzugt von 0,2 bis 30 Gew.-%, bezogen auf den Palladiumgehalt, besitzt und pro Nitrogruppen-Äquivalent 30 bis 6000, bevorzugt 50 bis 3000, besonders bevorzugt 80 bis 1000, ganz besonders bevorzugt 100 bis 300 Äquivalente Wasserstoff dem Katalysator zugeführt werden.

Die Erfindung betrifft weiterhin einen Katalysator mit Graphit oder graphithaltigem Koks als Träger, der eine BET-Oberfläche von 0,2-10 m²/g hat, mit einem durch Imprägnierung aufgebrachten Palladiumgehalt von 0,001 bis 7 Gew.-%, bezogen auf das Gesamtgewicht des Katalysators, und einem Bleigehalt von 0,1 bis 50 Gew.-%, bevorzugt von 0,2 bis 30 Gew%, bezogen auf den Palladiumgehalt.

Als Trägermaterial zur Herstellung der Katalysatoren werden graphithaltige Materialien eingesetzt. Dies sind die Graphite selbst, z.B. Elektrographit und Kokse, wie Nadelkoks oder Petrolkoks. Diese Träger haben BET-Oberflächen von 0,2-10 m²/g.

Der Katalysator wird hergestellt, indem Palladium und Blei getrennt oder gemeinsam in 1 bis 50, bevorzugt 2 bis 30, besonders bevorzugt 4 bis 10 Tränkschritten auf den Träger aufgebracht wird, zwischen denen jeweils eine Trocknung des Katalysatorträgers in einem warmen Gasstrom, bevorzugt Luft- oder Stickstoff-Strom, erfolgt. Zwischen Palladium- und Blei-Tränkungen kann eine Reduktion des Palladiums z.B. mit Wasserstoff erfolgen.

Zur Herstellung der erfindungsgemäßen Katalysatoren kann so vorgegangen werden, daß auf einen der genannten Träger in Form von Tabletten, Kugeln, Stranggranulat, Raschigringen, Pallringen, Wagenrädern, Wabenstrukturen von 1 bis 30 mm Abmessungen das Palladium in Form geeigneter Salze aufgetragen wird. Dazu können mehrere Tränkschritte ausgeführt werden, wobei nach jedem Auftrag getrocknet wird. Das Trocknen geschieht im Luftstrom bei Temperaturen von 30 bis 140°C, bevorzugt bei 30 bis 60°C, bevorzugt bei normalem Druck. Zum Tränken des Trägers können wäßrige und organische Lösungsmittel sowie deren Mischungen verwendet werden. Als Lösungsmittel können z.B. Wasser, NH₃, einfache Alkohole, Amine, Ketone, Ester, cyclische Ether, halogenierte KWs oder Nitrile eingesetzt werden. Beispiele für organische Lösungsmittel sind Methanol, Ethanol, Propanol, Isopropanol, Methylamin, Ethylamin, Isopropylamin, Aceton, Methyl-ethylketon, Dioxan, Methylenchlorid, Acetonitril und vergleichbare Lösungsmittel. Geeignete Palladiumsalze sind beispielsweise Palladiumchlorid, -nitrat, -acetylacetonat oder -acetat und ihre Amin-Komplexe. Bevorzugt wird halgeonfrei präpariert, dies bezieht sich auf das Lösungsmittel und das verwendete Metallsalz.

Es können organische und anorganische Bleiverbindungen eingesetzt werden. Geeignete organische Bleiverbindungen sind z.B. Tetraalkylblei wie z.B. Tetraethylblei. Geeignete Bleisalze sind beispielsweise Bleichlorid, Bleinitrat und Bleiacetat. Bevorzugt wird halogenfrei präpariert, das bezieht sich auf das Lösungsmittel und das verwendete Metallsalz. Nach dem Imprägnieren und dem abschließenden Trocknen steht der erfindungsgemäße Katalysator zur Verfügung.

Vor der ersten Inbetriebnahme wird der Katalysator aktiviert, indem er mit einem Wasserstoff-Strom von 1 bis 10 bar, bei Temperaturen von 250 bis 450°C, bevorzugt 300 bis 400°C, 1 bis 50 Stunden, bevorzugt 5 bis 30 Stunden behandelt wird.

Das erfindungsgemäße Hydrier-Verfahren wird bei einem Druck von 1 bis 30 bar, bevorzugt von 1 bis 15 bar, besonders bevorzugt von 1 bis 7 bar durchgeführt.

Das Nitroaromat und Wasserstoff enthaltende Eduktgasgemisch hat vor dem Katalysatorbett eine Temperatur von 200 bis 400°C, bevorzugt 230 bis 370°C, besonders bevorzugt 250 bis 350°C, die maximale Katalysatortemperatur beträgt 600°C, bevorzugt 550°C, besonders bevorzugt 500°C, ganz besonders bevorzugt. 460°C.

Der erfindungsgemäße Katalysator kann in Reaktoren ohne System zur Wärmeabfuhr eingesetzt werden.

Besonders bemerkenswert sind die geringen Verweilzeiten bzw. großen GHSVs bei dem erfindungsgemäßen Verfahren, das Katalysatorbelastungen von 0,05 bis 20 kg, bevorzugt von 0,1 bis 10 kg, Nitroaromat pro Liter Katalysator und Stunde zuläßt.

Das erfindungsgemäße Verfahren zeichnet sich besonders durch die Abwesenheit einer sog. Katalysator-Einfahrphase aus und liefert von Beginn an Selektivitäten oberhalb 99,7%, die nach kurzer Zeit 99,9% erreichen und überschreiten.

Ein weiterer wichtiger Vorteil des erfindungsgemäßen Verfahrens ist der quantitative Umsatz an Nitroaromat, der überraschenderweise und unerwartet auch bei kurzen Verweilzeiten bzw. hohen GHSVs erzielt wird.

Im erfindungsgemäßen Verfahren wird der Nitroaromat zu mehr als 99,95 %, bevorzugt mehr als 99,99 %, besonders bevorzugt mehr als 99,995 %, ganz besonders bevorzugt mehr als 99,999 % umgesetzt, das ist nicht einschränkend zu verstehen, da jeder beliebige Umsatzgrad eingestellt werden kann.

Die Katalysatoren können in jedem beliebigen Reaktor mit stationärem Katalysatorbett eingesetzt werden.

Eine technische Ausführung des Verfahrens kann wie folgt beschrieben werden:

Der Katalysator befindet sich stationär in einem adiabaten Reaktor bekannter Ausführungen (siehe z. B. Ullmanns, Enzyklopädie der technischen Chemie, 4. Aufl., Bd.3, S. 468-649; Kirk-Othmer, Encyclopedia of Chemical Technology, Vol.19 (1982), S. 880-914), das Katalysatorbett kann aber auch auf mehrere Reaktoren, die hintereinander oder parallel geschaltet sind, verteilt sein.

Dies können z. B. Reaktoren sein, wie sie für die Oxidation von Methanol zu Formaldehyd Stand der Technik sind.

Die Katalysatorschüttungen werden nach dem Stand der Technik auf oder zwischen gasdurchlässige Wandungen gebracht, wobei auf ausreichende Gasverteilung zu achten ist.

Der Katalysator kann statt in Schüttungen auch auf als Trägermaterial geeigneten Packungen präpariert und verwendet werden.

Vor der Katalysatorschüttung wird frischer Nitroaromat in den Kreisgasstrom, der hauptsächlich aus recycliertem und frisch zugesetztem Wasserstoff besteht, eindosiert. Bevorzugt wird jedoch der Nitroaromat im Frischwasserstoff vollständig verdampft und dann gasförmig in den Kreisgasstrom gegeben. Nach Durchgang durch das Katalysatorbett wird das Produktgas unter Dampfgewinnung abgekühlt, dies erfolgt mittels dem Fachmann bekannten Wärmetauschern. Danach wird das Produktgas abgekühlt, um aromatisches Amin und Reaktionswasser durch Kondensation aus dem Reaktionsgemisch zu entfernen. Das verbleibende Kreisgas wird, nach Abzweigen einer geringen Gasmenge zum Ausschleusen von gasförmigen Komponenten im Kreisgas, recycliert. Vor der Recyclierung muß das Kreisgas auf Eingangstemperatur vorgewärmt werden und frisches Edukt zugemischt erhalten.

Die Ausführungen zur Verfahrensweise sind prinzipieller Art und sollen nicht einschränkend wirken oder gewertet werden.

Das Verfahren eignet sich besonders, um Nitrobenzol oder Nitrotoluol zu hydrieren.

Das erfindungsgemäße Verfahren erlaubt große Katalysatorbelastungen bzw. GHSVs, die über denen des Standes der Technik liegen. Dabei werden auch zu Beginn der Umsetzung mit den erfindungsgemäßen Katalysatoren Selektivitäten größer 99,7 % bei vollständigem Umsatz erzielt.

Die Katalysatoren zeigen die größten bisher beschriebenen Selektivitäten bei der Gasphasenhydrierung von Nitroaromaten und gestatten, gegebenenfalls auf eine destillative Aufarbeitung des kondensierten Anilines zu verzichten.

### Beispiele

GHSV (**G**as **H**ourly **S**pace **V**elocity) gibt die stündliche Raumgeschwindigkeit des Gases unter Normalbedingungen bzw. beim angegeben Druck bezogen auf das Leervolumen an, das die Katalysatorschüttung einnimmt.

### Katalysatorpräparation

Als Trägermaterial wurde Graphitgranulat EG 17 der Firma Ringsdorff benutzt, das eine BET-Oberfläche von ca. 0,4-0,8 m²/g besitzt.
Die Korngrößen lagen zwischen 1 und 3 mm.

Die Beispiele sollen in keiner Weise einschränkend verstanden werden, auch mit anderen Graphiten und graphithaltigen Materialien mit geringer BET-Oberfläche werden ähnliche Ergebnisse erzielt.

Die Katalysatorpräparationen erfolgten in folgender Weise:

Graphitgranulat EG 17 der Firma Ringsdorf (Granulat 1-3 mm, Rüttelgewicht 650-1000 g/ L) mit einer Saugfähigkeit von 7 ml Acetonitril pro 100 g Träger wird in einem drehbaren Gefäß vorgelegt und unter Drehen mit einer Lösung aus Palladiumacetat in Acetonitril versetzt. Die Mischung wird bis zur vollständigen Aufnahme der Lösung durch den Träger bewegt. Darauf folgt eine 5 minütige Trocknung des Feststoffes in einem starken aufsteigenden 40°C warmen Luftstrom. Die Schritte Tränken und Trocknen werden so oft wiederholt, bis die gewünschte Menge an Palladium aufgebracht ist.

Darauf folgend werden Tränkungen und Trocknungen in gleicher Weise mit wässrigen Bleiacetat-tri-hydrat-Lösungen durchgeführt, bis die gewünschte Menge an Blei aufgebracht ist.

Der getrocknete Katalysator wird anschließend in einem heißen Wasserstoffstrom bei Normaldruck aktiviert.

### Beispiel 1

Kat 1 (2% Pd, 0,5% Pb auf EG17)
2000 g Träger
9 Tränkungen mit je
9,25 g PdAc₂ in 140 g Acetonitril
2 Tränkungen mit je
18 g PbAc₂ x 3 H₂O in 130 g Wasser
20 h bei 370°C aktiviert

### Beispiel 2

Kat 2 (Vergleichskat. ohne Bei, 2% Pd auf EG17)
2000 g Träger
9 Tränkungen mit je
9,25 g PdAc₂ in 140 g Acetonitril
20 h bei 370°C aktiviert

### Beispiel 3

In einem sehr gut isolierten Reaktor wurden 220 ml (219,0 g) des in Beispiel 1 hergestellten Katalysators mit 2,0 % Pd und 0,5 % Pb mit einer Schütthöhe von 180 mm eingebracht. Der Reaktor wurde am oberen Ende mit einem Verdampfer und Überhitzer versehen. Für die kontinuierliche Abführung des Produktgases war am Reaktorausgang ein gut isoliertes Rohr angeschlossen, das das Produkt zwecks Kondensation in ein System aus Rohrbündel- und Schlangenkühler leitete. Die Temperatur vor, in und nach der Katalysatorbettschüttung wurde mittels eines verschiebbaren Thermoelementes gemessen. Der Katalysator wurde zunächst unter Wasserstoffzufuhr über Verdampfer und Überhitzer bei Normaldruck im Reaktor bei 200°C 10 h behandelt. Danach wurde der Wasserstoffstrom auf 1620 l/h eingestellt. Bei Starttemperaturen von Tein = 202°C wurden 110 g/h Nitrobenzol mittels einer Dosierpumpe über den Verdampfer-Überhitzer in den Wasserstoffstrom eindosiert; das entsprach einem molaren Wasserstoff-Nitrobenzol-Verhältnis von 81 zu 1, was bei quantitativem Umsatz unter adiabaten Bedingungen zu einer Temperaturdifferenz zwischen Edukt- und Produkt-Gasstrom von ca. 200°C führen würde. Nach wenigen Stunden stellte sich ein Temperaturprofil im Katalysatorbett ein, das einem Wärmeverlust über die Reaktorwand von ca. 10% entsprach. Der Rest der Reaktionswärme verläßt das Katalysatorbett mit dem Produktgasgemisch. Die gaschromatographische Analyse des Kondensats ergab die in der Tabelle angegebenen Gehalte. Nach 3000 h zeigte der Katalysator keinerlei Anzeichen von Desaktivierung.
GHSV = 7460 h⁻¹

| Laufzeit h | Kat.-Nr. | NBz ppm | Selektivität % | Tein °C | Tmax °C |
|---|---|---|---|---|---|
| 6 | 1 | 0 | 99,88 | 202 | 385 |
| 129 | | 0 | 99,90 | 202 | 385 |
| 983 | | 0 | 99,94 | 202 | 386 |
| 1804 | | 0 | 99,93 | 202 | 383 |
| 2912 | | 0 | 99,95 | 202 | 384 |

Eine durchgehend hohe Selektivität von über 99,85 % gestattet, das das kondensierte Anilin ohne weitere Aufarbeitung zu verwenden, gegebenenfalls kann die Reinheit durch Partialkondensation weiter erhöht werden, wodurch die Leicht- und Hochsiederanteile von weniger als 0,12 % weiter verringert werden.

Damit ist es möglich, eine kostspielige destillative Aufarbeitung zu umgehen.

### Beispiel 4 (Vergleichsbeispiel mit bleifreiem Katalysator)

Analog zu Beispiel 3 wurden in denselben Reaktor 220 ml (223 g) Katalysator mit 2,0 % Pd aus Beispiel 2 eingefüllt. Nach zu Beispiel 3 analoger Wasserstoff-Behandlung wurden bei Starttemperaturen von Tein = 201°C mit der Dosierung von Nitrobenzol begonnen. Nach 40 h wurde bei quantitativem Umsatz eine Anilin-Selektivität von 99,5 % erreicht. Nach 170 h erhöhte sich die Selektivität für Anilin auf mehr als 99,6 %. Nach 1000 h zeigte der Katalysator keine Anzeichen einer beginnenden Desaktivierung.
GHSV = 7460 h⁻¹

| Laufzeit h | Kat.-Nr. | NBz ppm | Selektivität % | Tein °C | Tmax °C |
|---|---|---|---|---|---|
| 40 | 2 | 0 | 99,49 | 207 | 376 |
| 214 | | 0 | 99,63 | 206 | 375 |
| 1004 | | 0 | 99,73 | 207 | 377 |

Der Kontakt zeigt unter sonst identischen Bedingungen über 400 % mehr Nebenprodukte als der Katalysator in Beispiel 3.

### Beispiel 5 (Vergleichsbeispiel, DE 2849002)

Analog zu Beispiel 3 wurden in denselben Reaktor 220 ml eines Katalysators analog präpariert nach DE 2849002, Beispiel 1 der Zusammensetzung 9 g Pd, 9 g V, 3 g Pb auf α-Aluminiumoxid (SPH 512 der Fa. *Rhône-Poulenc*) eingefüllt. Nach Aktivierung und unter Versuchsbedingungen lt. Beispiel 3 wurden folgende Ergebnisse erhalten:
GHSV = 7460 h⁻¹

| Laufzeit h | NBz ppm | Selektivität % | Tein °C | Tmax °C |
|---|---|---|---|---|
| 3,5 | 0 | 98,8 | 199 | 383 |
| 90 | 0 | 99,1 | 195 | 372 |
| 160 | 100 | 99,6 | 200 | 375 |

Der Katalysator zeigt in einem ölthermostatisierten Rohrreaktor bei gleicher Nitrobenzolbelastung und einem Wasserstoff-Nitrobenzol-Verhältnis von 6/1 (GHSV = 637 h⁻¹) eine Standzeit von ca. 1000 h und eine Selektivität van ca. 99,8% gemittelt über den Umsetzungszyklus.

Für eine Fahrweise mit großem Wasserstoffüberschuß und großer Nitrobenzolbelastung ist der Katalysator ungeeignet. Die Selektivitäten sind deutlich niedriger als bei erfindungsgemäßen Verfahren.

Folgende Beispiele 6 und 7 mit ca. 2 L Katalysator 1 wurden in ölthermostatisierten Wärmetauscherrohren aus V2A mit einer für technische Reaktoren üblichen Länge von ca. 300 cm und einem Innendurchmesser von ca. 3 cm bei Normaldruck durchgeführt.

Die Belastung betrug 0,65 g/ml h, die Temperatur des Wärmeträgers wurde auf 250°C eingestellt.

### Beispiel 6

GHSV = 9707 h⁻¹
H2/NBz = 81/1 Standzeit > 5000 h (kein Nitrobenzoldurchbruch)

### Beispiel 7 (Vergleichsbeispiel mit geringem Wasserstoffüberschuß)

GHSV = 2131 h⁻¹
H2/NBz = 17/1 Standzeit 108 h (Nitrobenzoldurchbruch > 100ppm)

Trotz deutlich geringerer Gasbelastung bzw. GHSV desaktiviert der Vergleichsversuch deutlich schneller. Unterhalb eines kritischen Wasserstoff- zu Nitrobenzol-Verhältnisses findet eine deutlich voranschreitende Katalysatordesaktivierung statt.

## Patentansprüche

1. Verfahren zur Herstellung aromatischer Amine der Formel 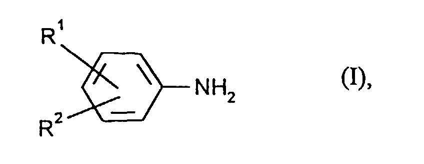 in der
R¹ und R² unabhängig voneinander Wasserstoff, Methyl oder Ethyl bedeuten, wobei R¹ zusätzlich Amino bedeuten kann,
durch Hydrierung von Nitroaromaten der Formel 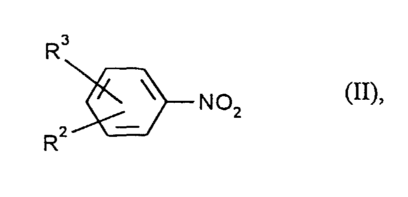 in der
R² und R³ unabhängig voneinander Wasserstoff, Methyl oder Ethyl bedeuten, wobei R³ zusätzlich Nitro bedeuten kann,
mit Wasserstoff an ortsfesten angeordneten Katalysatoren in der Gasphase, wobei als Katalysator Palladium und Blei auf Graphit oder graphithaltigem Koks als Träger, der eine BET-Oberfläche von 0,2-10 m²/g hat, eingesetzt wird, dadurch gekennzeichnet, daß der Katalysator einen Palladiumgehalt von 0,001 bis 7 Gew.-%, bezogen auf das Gesamtgewicht des Katalysators, besitzt und einen Bleigehalt von 0,1 bis 50 Gew.-%, bezogen auf den Palladiumgehalt, aufweist und pro Nitrogruppen-Äquivalent 30 bis 6000 Äquivalente Wasserstoff dem Katalysator zugeführt werden.

2. Verfahren nach Anspruch 1, dadurch gekennzeichnet, daß bei einem Druck von 1 bis 30 bar gearbeitet wird.

3. Verfahren nach Anspruch 1, dadurch gekennzeichnet, daß das Nitroaromat- und Wasserstoff-enthaltende Eduktgasgemisch vor dem Katalysatorbett eine Temperatur von 200 bis 400°C hat und die maximale Katalysatortemperatur 600°C beträgt.

4. Verfahren nach Anspruch 1, dadurch gekennzeichnet, daß eine Katalysatorbelastung von 0,05 bis 20 kg Nitroaromat pro Liter Katalysator und Stunde eingestellt wird.

5. Verfahren nach Anspruch 1, dadurch gekennzeichnet, daß die Umsetzung in Reaktoren ohne System zur Abfuhr der Reaktionswärme durchgeführt wird.

6. Verfahren nach Anspruch 1, dadurch gekennzeichnet, daß Nitrobenzol oder Nitrotoluol hydriert wird.

7. Katalysator, insbesondere für die Hydrierung von Nitroaromaten, mit Graphit oder graphithaltigem Koks als Träger, der eine BET-Oberfläche von 0,2-10 m²/g hat, mit einem durch Imprägnierung aufgebrachten Palladiumgehalt von 0,001 bis 7 Gew.-%, bezogen auf das Gesamtgewicht des Katalysators, und einem Bleigehalt von 0,1 bis 50 Gew.-%, bezogen auf den Palladiumgehalt.

8. Katalysator nach Anspruch 7, dadurch gekennzeichnet, daß das Palladium in 1 bis 50 Tränkschritten auf den Träger aufgebracht wird, zwischen denen jeweils eine Trocknung des Katalysatorträgers in einem warmen Gasstrom erfolgt.

9. Katalysator nach Ansprüchen 7 und 8, dadurch gekennzeichnet, daß der Katalysator nach den Tränkungen und vor dem Einsatz in einem Wasserstoff-Strom bei 1 bis 10 bar sowie bei Temperaturen von 250 bis 450°C aktiviert wird.

## Claims

1. Process for the manufacture of aromatic amines of the formula 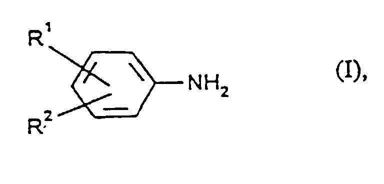 in which
R¹ and R² independently of each other stand for hydrogen, methyl or ethyl, wherein R¹ can in addition stand for amino,
by hydrogenation of nitroaromatics of the formula 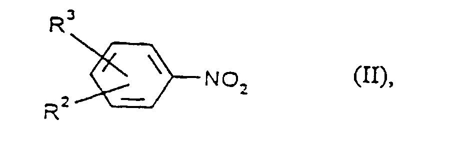 in which
R² and R³ independently of each other stand for hydrogen, methyl or ethyl, wherein R³ can in addition stand for nitro,
with hydrogen on stationary catalysts in the gas phase, wherein palladium and lead on graphite or graphite-containing coke as support, which has a BET surface area of 0.2 to 10 m²/g, is used as catalyst, characterized in that the catalyst has a palladium content of 0.001 to 7 wt.%, relative to the total weight of the catalyst, and a lead content of 0.1 to 50 wt.%, relative to the palladium content, and per equivalent of nitro groups 30 to 6000 equivalents hydrogen are fed to the catalyst.

2. Process according to claim 1, characterized that the operating pressure is 1 to 30 bar.

3. Process according to claim 1, characterized in that the educt gas mixture containing nitroaromatic and hydrogen has a temperature before the catalyst bed of 200 to 400 °C and the maximum catalyst temperature is 600 °C.

4. Process according to claim 1, characterized in that a catalyst loading of 0.05 to 20 kg nitroaromatic per litre of catalyst per hour is set.

5. Process according to claim 1, characterized in that the reaction is carried out in reactors without a system for dissipation of the heat of reaction.

6. Process according to claim 1, characterized in that nitrobenzene or nitrotoluene is hydrogenated.

7. Catalyst, in particular for the hydrogenation of nitroaromatics, with graphite or graphite-containing coke as support, which has a BET surface area of 0.2 to 10 m²/g, a palladium content, applied by impregnation, of 0.001 to 7 wt.%, relative to the total weight of the catalyst, and a lead content of 0.1 to 50 wt.%, relative to the palladium content.

8. Catalyst according to claim 7, characterized in that the palladium is applied to the support in 1 to 50 impregnation stages, between which in each case the catalyst support is dried in a hot gas stream.

9. Catalyst according to claims 7 and 8, characterized in that the catalyst, after the impregnations and before use, is activated in a hydrogen stream at 1 to 10 bar and at temperatures of 250 to 450 °C.

## Revendications

1. Procédé pour la préparation d'amines aromatiques de formule 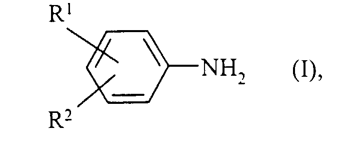 dans laquelle
R¹ et R² représentent chacun, indépendamment l'un de l'autre, l'hydrogène, un groupe méthyle ou éthyle, R¹ pouvant en outre représenter un goupe amino, par hydrogénation de dérivés nitroaromatiques de formule 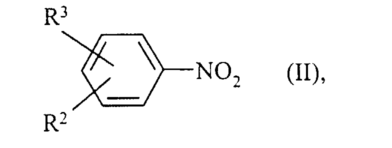 dans laquelle
R² et R³ représentent chacun, indépendamment l'un de l'autre, l'hydrogène, un groupe méthyle ou éthyle, R³ pouvant en outre représenter un groupe nitro, sur des catalyseurs en disposition fixe, en phase gazeuse, dans lequel on utilise des catalyseurs consistant en palladium et plomb sur un support consistant en graphite ou coke graphitique à une surface BET de 0,2 à 10 m²/g, ce procédé se caractérisant en ce que le catalyseur a une teneur en palladium de 0,001 à 7 % en poids, par rapport à son poids total, et une teneur en plomb de 0,1 à 50 % en poids par rapport à la teneur en palladium, et en ce que l'on envoie sur le catalyseur de 30 à 6000 équivalents d'hydrogène par équivalent de groupe nitro.

2. Procédé selon la revendication 1, caractérisé en ce que l'on opère à une pression de 1 à 30 bar.

3. Procédé selon la revendication 1, caractérisé en ce que le mélange gazeux de départ contenant le dérivé nitroaromatique et l'hydrogène est à une température de 200 à 400°C avant le lit de catalyseur et en ce que la température maximale du catalyseur est de 600°C.

4. Procédé selon la revendication 1, caractérisé en ce que l'on règle à une charge du catalyseur de 0,05 à 20 kg de dérivé nitroaromatique par litre de catalyseur et par heure.

5. Procédé selon la revendication 1, caractérisé en ce que la réaction est réalisée dans des réacteurs non équipés de système d'évacuation de la chaleur de réaction.

6. Procédé selon la revendication 1, caractérisé en ce que l'on hydrogène du nitrobenzène ou du nitrotoluène.

7. Catalyseur, convenant en particulier pour l'hyrogénation de dérivés nitroaromatiques, dont le support consiste en graphite ou coke graphitique, à une surface BET de 0,2 à 10 m²/g, à une teneur en palladium, appliqué par imprégnation, de 0,001 à 7 % en poids, par rapport à son poids total, et une teneur en plomb de 0,1 à 50 % en poids, par rapport à la teneur en palladium.

8. Catalyseur selon la revendication 7, caractérisé en ce que le palladium est appliqué sur le support en 1 à 50 opérations d'imprégnation entre lesquelles on sèche le support de catalyseur dans un courant de gaz chaud.

9. Catalyseur selon les revendications 7 et 8, caractérisé en ce que, après les imprégnations et avant l'utilisation, le catalyseur est activé dans un courant d'hydrogène de 1 à 10 bar à des températures de 250 à 450°C.
